# EUROPÄISCHE PATENTSCHRIFT

(11) **EP 1 445 020 B1**
(45) Veröffentlichungstag und Bekanntmachung des Hinweises auf die Patenterteilung: **28.02.2007**
(21) Anmeldenummer: 04001158.7
(22) Anmeldetag: 21.01.2004
(51) Int. Cl.: B01L 3/00, G01N 33/558, C12Q 1/00

(54) **Analytisches Testelement und Verfahren für Blutuntersuchungen**
Analytical test element and blood test method
Element d'essai et procédé pour test sanguin

(30) Priorität: 07.02.2003 DE 10305050
(43) Veröffentlichungstag der Anmeldung: 11.08.2004
(73) Patentinhaber: Roche Diagnostics GmbH, 68305 Mannheim (DE); F. HOFFMANN-LA ROCHE AG, 4070 Basel (CH)
(72) Erfinder: Noetzel, Siegfried, Dr., 69259 Wilhelmsfeld (DE); Bogardi, Jean-Philippe, Dr., 8010 Graz (AT); Mangold, Dieter, Dr., 67133 Maxdorf (DE)
(74) Vertreter: Pfiz, Thomas

(56) Entgegenhaltungen:
- WO-A-01/24931
- WO-A-99/18436
- DE-A- 4 323 672
- US-A1- 2003 003 522

## Beschreibung

Die Erfindung betrifft ein analytisches Testelement für Blutuntersuchungen, insbesondere durch einen Einmal-Schnelltest, mit einem eine vorzugsweise mikrofluidische Kanalstruktur zum Fließtransport einer Blutprobe von einer Aufgabestelle zu mindestens einer Untersuchungsstelle aufweisenden Substratkörper. Die Erfindung betrifft weiter ein entsprechendes Verfahren zur Durchführung von Blutuntersuchungen bei welchem eine Blutprobe in einem analytischen Testelement über eine Kanalstruktur von einer Aufgabestelle zu mindestens einer Untersuchungsstelle geleitet wird.

Ein Testelement dieser Art ist aus der WO 01/24931 bekannt. Dort wird speziell zur Separation von Plasma oder Serum aus einer Vollblutprobe eine Kanal- bzw. Fließstruktur bestehend aus zwei kapillaraktiven Zonen beschrieben, wobei eine erste Zone aus einem porösen Matrixmaterial besteht und eine damit in Kontakt stehende zweite Zone einen oder mehrere Kapillarkanäle umfasst. Damit soll das in der ersten Zone gewonnene Plasma als Zielflüssigkeit beispielsweise für Glucosetests in der zweiten Zone frei von interferierenden Bestandteilen bereitgestellt werden.

Allgemein kann unter einem Testelement ein trägergebundenes fluidisches (Mikro)System zur Aufnahme einer Flüssigprobe verstanden werden, welches unabhängig von einer Laborumgebung eine Probenaufbereitung für eine sofortige oder spätere Analyse ermöglicht. Solche Testelemente sind in der Regel als Einmalartikel bzw. Disposable für eine patientennahe Diagnostik bestimmt, wobei alle für die Testdurchführung erforderlichen Reagenzien auf dem Träger bzw. Bauteil bereitgestellt werden, so dass ohne besonderen Handhabungsaufwand eine Anwendung auch durch Laien möglich ist.

Solche Testelemente werden als Teststreifen insbesondere zur Blutglucoseüberwachung von Diabetikern eingesetzt. Demgegenüber erlaubt die Bestimmung von Hämo-globin A1c eine retrospektive Abschätzung der mittleren Glucosekonzentration der letzten Wochen und damit der Güte der Stoffwechseleinstellung des Diabetikers. HbA1c ist definiert als Hämoglobin A, das an den N-terminalen Valinresten der β-Ketten mit Glucose glykiert wurde. HbA1c wird üblicherweise als Prozentwert des Gesamthämoglobins angegeben, wofür neben dem HbA1 c-Gehalt auch die Hämoglobinkonzentration aus der gleichen Blutprobe bestimmt werden muss. Diese Doppelbestimmung von Hb und HbA1c wird bisher in Laborgeräten durchgeführt, die in der Handhabung sehr komplex und damit fehleranfällig sind bzw. hohe Kosten verursachen.

Ausgehend hiervon liegt der Erfindung die Aufgabe zugrunde, die im Stand der Technik aufgetretenen Nachteile zu vermeiden und ein Testelement dahin zu verbessern, dass mit möglichst wenig Anwenderinteraktion und geringem Reagenzienaufwand Bluttests speziell bei hoher Konzentration des Analyten in der Ausgangsprobe kostengünstig möglich sind.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Dementsprechend wird erfindungsgemäß vorgeschlagen, dass die Kanalstruktur einen mit der Blutprobe beaufschlagbaren, mit Trennmitteln zur Rückhaltung von korpuskulären Blutbestandteilen versehenen Verdünnungskanal und einen an einer Mischstelle mit dem Verdünnungskanal zusammengeführten, ein zu verdünnendes Blutprobenaliquot zuleitenden Probenkanal aufweist. Damit ist es möglich, vom Anwender aufgebrachtes Vollblut mit eigenen Flüssigbestandteilen zu verdünnen, ohne dass zusätzliche Flüssigkeiten eingelagert werden müssten. Die Verdünnung mit Probenmaterial erfolgt dabei durch den Fließtransport selbstgesteuert, so dass keine komplizierten manuellen Handhabungsschritte durch den Anwender bzw. komplexe mechanische Einwirkungen durch ein Analysegerät erforderlich sind.

Vorteilhafterweise sind der Probenkanal und der Verdünnungskanal über einen Knotenpunkt unter paralleler Teilung des Probenflusses mit der Blutprobe beaufschlagbar. Die Blutprobe kann dabei auf einem zentralen Punkt aufgegeben und an dem Knotenpunkt bzw. an einer Verzweigung des Probenkanals in einem quantifizierten, vordefinierten Verhältnis geteilt werden, so dass Probenkanal und Verdünnungskanal mit der Probe beaufschlagbar sind. Zur Einstellung eines vorgegebenen Teilungsverhältnisses der hindurchgeleiteten Teilströme der Blutprobe ist es vorteilhaft, wenn die Kanalquerschnitte des Proben- und Verdünnungskanals entsprechend aufeinander abgestimmt sind. Dabei ist es speziell zur Reduzierung der Hämoglobinkonzentration günstig, wenn der Durchfluss durch den Verdünnungskanal mehr als das 10fache, vorzugsweise mehr als das 100fache des Durchflusses durch den Proben kanal beträgt.

Zur Rückhaltung der Zellbestandteile ist es vorteilhaft, wenn in dem Verdünnungskanal vorzugsweise in einer Filterkammer ein insbesondere durch ein Glasfaservlies oder eine mikroporöse Filtermatrix oder Filtermembran gebildetes Filterelement als Trennmittel angeordnet ist. Alternativ oder ergänzend kann der Verdünnungskanal eine zur Rückhaltung von Zellbestandteilen der Blutprobe ausgebildete Mikrostrukturgeometrie als Trennmittel besitzen.

Eine weitere vorteilhafte Ausführung sieht vor, dass die Mischstelle durch eine mit einem Lysemittel versehene Lysekammer zur Hämolyse der verdünnten Blutprobe gebildet ist.

Eine vorteilhafte Ausführung der Erfindung liegt darin, dass die Kanalstruktur einen ersten Analysekanal zur Bestimmung des Gesamthämoglobinwerts (Hb) der Blutprobe und einen zweiten Analysekanal zur Bestimmung eines Glykohämoglobinwerts (HbA1c) der Blutprobe aufweist. Damit kann ein HbA1c-Nachweis als 1-Schritt-Test durch eine einfache Blutauftragung auf einem Testelement in zugeordneten Fließpfaden realisiert werden.

Eine vorteilhafte Ausführung sieht vor, dass die Analysekanäle in paralleler Anordnung über eine Verzweigung als Stromteiler stromab von der Mischstelle mit der verdünnten Blutprobe beaufschlagbar sind.

Für die Gesamthämoglobinbestimmung ist es günstig, wenn der erste Analysekanal eine Oxidationskammer mit einem eingelagerten Oxidationsmittel, insbesondere Ferricyanid zur Oxidation von freigesetztem Hämoglobin aufweist.

Vorteilhafterweise ist der zweite Analysekanal zur immunturbidimetrischen Bestimmung der Glykohämoglobinkonzentration ausgebildet. Dies lässt sich vorteilhaft dadurch realisieren, dass der zweite Analysekanal eine erste Reaktionskammer mit darin dispensierten HbA1c-Antikörpern und eine der ersten Reaktionskammer nachgeordnete zweite Reaktionskammer mit einem eingelagerten Agglutinator aufweist.

Dem Fachmann sind beispielsweise aus EP-A-0 989 407 auch weitere prinzipielle Methoden zur Bestimmung von HbA1c aus Blut bekannt. Diese Methoden können ebenfalls in der vorliegenden Erfindung Anwendung finden.

Die Endabschnitte der Analysekanäle sind als Küvetten für eine photometrische Untersuchung ausgebildet und bilden somit Untersuchungsstellen für einen einfachen, berührungslosen Nachweis.

Um die untersuchte Probenflüssigkeit sicher und hygienisch aufzufangen, ist es vorteilhaft, wenn die Analysekanäle in ein Auffangreservoir münden.

Ein selbsttätiger Fließtransport lässt sich dadurch realisieren, dass die Kanalstruktur ganz oder teilweise eine Kapillargeometrie aufweist. Zur Steuerung des Fließtransports ist es von Vorteil, wenn die Kanalstruktur, beispielsweise durch Oberflächenbehandlung, Plasmabehandlung oder Beschichtung, modifizierte Wandbereiche aufweist. Eine weitere vorteilhafte Ausführung sieht vor, dass die Kanalstruktur insbesondere durch hydrophile oder hydrophobe Kanalabschnitte gebildete Ventilelemente zur Steuerung des Fließtransports aufweist. Grundsätzlich ist es auch möglich, dass der Fließtransport in der Kanalstruktur durch auf den Substratkörper einwirkende äußere Steuerungsmittel, insbesondere lokale Druckbeaufschlagung oder Zentrifugalkräfte extern steuerbar ist.

In verfahrensmäßiger Hinsicht wird die eingangs genannte Aufgabe dadurch gelöst, dass aus der Blutprobe Flüssigbestandteile gewonnen und in einen zu analysierenden Teil der Blutprobe zu dessen Verdünnung eingebracht werden. Eine vorteilhafte Ausgestaltung sieht hier vor, dass eine Vollblutprobe als Ausgangsmaterial in parallelen Teilströmen in einen Verdünnungskanal und einen Probenkanal der Kanalstruktur eingespeist wird, und dass der in dem Verdünnungskanal von Zellbestandteilen abgereicherte Teilstrom an einer Mischstelle mit dem Teilstrom in dem Probenkanal zusammengeführt wird.

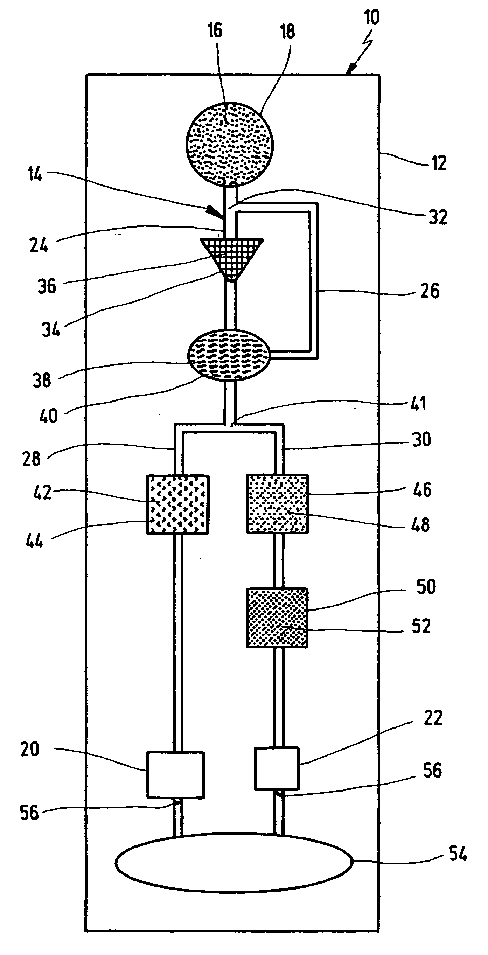

Im Folgenden wird die Erfindung anhand eines in der Zeichnung in schematischer Weise dargestellten Ausführungsbeispiels näher erläutert. Die einzige Figur zeigt ein analytisches Testelement zur Bestimmung der Hb- und HbA1c-Werte einer Blutprobe in einem Schnelltest.

Das Testelement 10 umfasst einen langgestreckten Träger- bzw. Substratkörper 12 mit einer darin ausgebildeten Kanalstruktur 14 zum Fließtransport mikroskopischer Probenmengen (µl) einer zu untersuchenden Blutprobe 16 von einer Aufgabezone 18 zu Mess- bzw. Untersuchungsstellen 20, 22 für Hb und HbA1c.

Der Substratkörper 12 kann als Spritzgussformteil aus Kunststoff oder als Verbundteil aus mehreren Folienlagen gebildet sein. Er ist als Verbrauchsmittel bzw. so genanntes "Disposable" für einen Einmaltest bestimmt.

Die Kanalstruktur 14 kann direkt in den Substratkörper eingeformt oder durch gesonderte Fertigungsschritte wie Prägen oder Stanzen ausgebildet sein. Sie weist zumindest abschnittweise eine geeignete Kapillargeometrie für einen selbsttätigen kapillaraktiven Fließtransport der Blutflüssigkeit auf.

Ausgehend von der Aufgabezone 18 umfasst die Kanalstruktur 14 einen Verdünnungskanal 24, einen Aliquot- bzw. Probenkanal 26 sowie zwei zu den Untersuchungsstellen 20, 22 führende Analysekanäle 28, 30.

Der Verdünnungskanal 24 und der Probenkanal 26 sind über einen Knotenpunkt 32 unter paralleler Aufspaltung des Probenflusses mit der Blutprobe 16 beaufschlagbar. Durch eine entsprechende Ausgestaltung der Kanalquerschnitte beträgt der Durchfluss durch den Verdünnungskanal 24 ein Vielfaches des Durchflusses durch den Probenkanal 26.

In dem Verdünnungskanal 24 ist in einer Trennkammer 34 ein Trennmittel 36 zur Rückhaltung von Zellbestandteilen des hindurchfließenden Teils der Blutprobe 16 auf. Solche Trennmittel 36 können beispielsweise durch ein in der Trennkammer 34 angeordnetes Glasfaservlies gebildet sein.

Der Verdünnungskanal 24 und der Probenkanal 26 münden in einer mit einem Lysemittel 38 versehenen Misch- bzw. Lysekammer 40. Diese kommuniziert auslassseitig über eine Verzweigung 41 als Stromteiler mit den Analysekanälen 28, 30.

In dem ersten Analysekanal 28 ist zur Oxidation von freigesetztem Hämoglobin eine mit Oxidationsreagenz 42 wie Kaliumhexacyanoferrat versehene Oxidationskammer 44 angeordnet. Stromab davon ist die Untersuchungsstelle 20 als Küvette für eine photometrische Hb-Bestimmung ausgebildet.

Der zweite Analysekanal 30 dient zur immunturbidimetrischen Bestimmung von glykierten Hämoglobinen. Er weist zu diesem Zweck eine erste Reaktionskammer 46 mit darin dispensierten HbA1c-Antikörpern 48 und eine nachgeordnete zweite Reaktionskammer 50 mit einem eingelagerten Agglutinationsmittel 52 für überschüssige HbA1c-Antikörper auf. Stromab davon ist die zweite Untersuchungsstelle 22 ebenfalls als Küvette für eine photometrische Trübungsmessung ausgebildet.

Beide Analysekanäle 20, 22 münden in ein gemeinsames Auffangreservoir 54 als "Waste" für die untersuchten Flüssigproben. Zur Steuerung des Fließtransports können ausgangsseitig der Küvetten 20, 22 und gegebenenfalls der Kammern 40,44,46,50 beispielsweise durch hydrophile oder hydrophobe Oberflächenmodifikation gebildete Sperren oder Ventilelemente 56 angeordnet sein. Diese Elemente ermöglichen die Steuerung von Reaktionsabläufen, insbesondere die Kontrolle des Probenvolumens und des Messablaufs, das Auflösen eingelagerter Trockenreagenzien und deren Durchmischung in den Reaktionskammern, z.B. durch temporäre Unterbrechung des Flüssigkeitsstroms.

Zur Durchführung eines in-vitro-Schnelltests zur Bestimmung von glykiertem Hämoglobin wird von einem Anwender eine geringe Menge an Vollblut auf die Aufgabezone 18 aufgetragen. Ein Aliquot davon wird über den Probenkanal 26 in die Lysekammer 40 geleitet und dort mit dem durch Erythrozytenabreicherung in dem Verdünnungskanal 24 gewonnenen Plasma gemischt. Dadurch wird eine definierte Verdünnung bzw. Reduzierung der Analytkonzentration erreicht, ohne dass zusätzliche Verdünnungsflüssigkeiten verarbeitet werden müssten. Zugleich erfolgt in der Lysekammer 40 eine Durchmischung der Probe mit dem als Trockensubstanz vorgehaltenen Lysemittel 38 (beispielsweise Saponin), wodurch eine Lyse der Erythrozyten unter Freisetzung des rotpigmentierten Hämoglobins herbeigeführt wird.

Ein Teil des verdünnten Hämolysats wird über die Verzweigung 41 in den ersten Analysekanal 28 geleitet und in der Oxidationskammer 44 in ein Derivat mit charakteristischem Spektrum überführt. Nach Einleitung in die Küvette 20 kann die Gesamthämoglobinkonzentration Hb mittels eines nicht gezeigten Photometers gemessen werden.

Zur Glykohämoglobinbestimmung wird ein weiterer Anteil des verdünnten Hämolysats über die Verzweigung 41 in den zweiten Analysekanal 30 geleitet. Dort wird in der ersten Reaktionskammer das Glykohämoglobin HbA1c aus der Probe mit den im Überschuss zugemischten HbA1c-Antikörpern 48 in einen löslichen Antigen-Antikörper-Komplex überführt. Die restlichen freien Antikörper 48 werden in der zweiten Reaktionskammer 50 agglutiniert und anschließend in der Küvette 22 turbidimetrische gemessen. Die Trübungsänderung verhält sich dabei umgekehrt proportional zur Menge des gebundenen Glykohämoglobins. Das endgültige Ergebnis wird schließlich als Verhältnis zwischen HbA1c und Hb errechnet.

Der vorstehend beschriebene Testablauf ermöglicht eine 1-Schritt-Handhabung ohne zusätzliche Einlagerung oder Eindosierung von Flüssigkeiten. Es versteht sich, dass durch die gesteuerte Auflösung dispensierter und getrockneter Reagenzien in selbstgesteuerten mikrofluidischen Reaktionspfaden auch andere Ausführungen eines HbA1c-Tests möglich sind, unter anderem auch unter Anwendung anderer Bestimmungsmethoden, wobei gewonnenes Plasmavolumen auch in späteren Prozessschritten z.B. zum Auswaschen überschüssiger Reagenzien genutzt werden kann. Grundsätzlich ist es möglich, dass die Hb- und HbA1c-Bestimmung in seriell angeordneten Kanalabschnitten erfolgt. Denkbar ist es auch, dass zumindest ein Teil der Kanalstruktur durch ein poröses Matrixmaterial realisiert wird.

## Patentansprüche

1. Analytisches Testelement für Blutuntersuchungen, insbesondere durch einen Einmal-Schnelltest, mit einem eine, vorzugsweise mikrofluidische, Kanalstruktur (14) zum Fließtransport einer Blutprobe von einer Aufgabestelle (18) zu mindestens einer Untersuchungsstelle (20,22) aufweisenden Substratkörper (12), **dadurch gekennzeichnet, dass** die Kanalstruktur (14) einen mit der Blutprobe beaufschlagbaren, mit Trennmitteln (36) zur Rückhaltung von korpuskulären Blutbestandteilen versehenen Verdünnungskanal (24) und einen an einer Mischstelle (40) mit dem Verdünnungskanal (24) zusammengeführten, ein zu verdünnendes Blutprobenaliquot zuleitenden Probenkanal (26) aufweist.

2. Analytisches Testelement nach Anspruch 1, **dadurch gekennzeichnet, dass** der Probenkanal (26) und der Verdünnungskanal (24) über einen Knotenpunkt (32) unter paralleler Teilung des Probenflusses mit der Blutprobe beaufschlagbar sind.

3. Analytisches Testelement nach Anspruch 1 oder 2, **dadurch gekennzeichnet, dass** die Kanalquerschnitte des Proben- und Verdünnungskanals (26,24) zur Einstellung eines vorgegebenen Teilungsverhältnisses der hindurchgeleiteten Teilströme der Blutprobe aufeinander abgestimmt sind.

4. Analytisches Testelement nach einem der Ansprüche 1 bis 3, **dadurch gekennzeichnet, dass** der Durchfluss durch den Verdünnungskanal (24) mehr als das 10fache, vorzugsweise mehr als das 100fache des Durchflusses durch den Probenkanal (26) beträgt.

5. Analytisches Testelement nach einem der Ansprüche 1 bis 4, **dadurch gekennzeichnet, dass** in dem Verdünnungskanal (24) vorzugsweise in einer Filterkammer (34) ein insbesondere durch ein Glasfaservlies oder eine mikroporöse Filtermatrix oder Filtermembran gebildetes Filterelement als Trennmittel (36) angeordnet ist.

6. Analytisches Testelement nach einem der Ansprüche 1 bis 5, **dadurch gekennzeichnet, dass** der Verdünnungskanal (24) eine zur Rückhaltung von Zellbestandteilen der Blutprobe ausgebildete Mikrostrukturgeometrie als Trennmittel (36) besitzt.

7. Analytisches Testelement nach einem der Ansprüche 1 bis 6, **dadurch gekennzeichnet, dass** die Mischstelle (40) durch eine mit einem Lysemittel (38) versehene Lysekammer (40) zur Hämolyse der verdünnten Blutprobe gebildet ist.

8. Analytisches Testelement nach einem der Ansprüche 1 bis 7, **dadurch gekennzeichnet, dass** die Kanalstruktur (14) einen ersten Analysekanal (28) zur Bestimmung des Gesamthämoglobinwerts (Hb) der Blutprobe und einen zweiten Analysekanal (30) zur Bestimmung eines Glykohämoglobinwerts (HbA1c) der Blutprobe aufweist.

9. Analytisches. Testelement nach Anspruch 8, **dadurch gekennzeichnet, dass** die Analysekanäle (28,30) über eine Verzweigung (41) als Stromteiler stromab von der Mischstelle (40) mit der verdünnten Blutprobe beaufschlagbar sind.

10. Analytisches Testelement nach Anspruch 8 oder 9, **dadurch gekennzeichnet, dass** der erste Analysekanal (28) eine Oxidationskammer (44) mit einem eingelagerten Oxidationsmittel (42), insbesondere Ferricyanid zur Oxidation von freigesetztem Hämo-globin aufweist.

11. Analytisches Testelement nach einem der Ansprüche 8 bis 10, **dadurch gekennzeichnet, dass** der zweite Analysekanal (30) zur immunturbidimetrischen Bestimmung der Glykohämoglobinkonzentration ausgebildet ist.

12. Analytisches Testelement nach einem der Ansprüche 8 bis 11, **dadurch gekennzeichnet, dass** der zweite Analysekanal (30) eine erste Reaktionskammer (46) mit darin dispensierten HbA1c-Antikörpern (48) zur Bildung von löslichen Antigen-Antikörper-Komplexen mit dem Glykohämoglobin aus der Blutprobe aufweist.

13. Analytisches Testelement nach Anspruch 12, **dadurch gekennzeichnet, dass** der zweite Analysekanal (30) eine der ersten Reaktionskammer nachgeordnete zweite Reaktionskammer(50) mit einem eingelagerten Agglutinator (52) zur Bildung von unlöslichen Immunkomplexen mit überschüssigen HbA1c-Antikörpern aufweist.

14. Analytisches Testelement nach einem der Ansprüche 8 bis 13, **dadurch gekennzeichnet, dass** als Küvetten für eine photometrische Untersuchung ausgebildete Endabschnitte der Analysekanäle (28,30) jeweils eine Untersuchungsstelle (20,22) bilden.

15. Analytisches Testelement nach einem der Ansprüche 8 bis 14, **dadurch gekennzeichnet, dass** die Analysekanäle (28,30) in ein Auffangreservoir (54) münden.

16. Analytisches Testelement nach einem der Ansprüche 1 bis 15, **dadurch gekennzeichnet, dass** die Kanalstruktur (14) zumindest abschnittsweise eine Kapillargeometrie für einen selbsttätigen kapillaraktiven Fließtransport aufweist.

17. Analytisches Testelement nach einem der Ansprüche 1 bis 16, **dadurch gekennzeichnet, dass** die Kanalstruktur (14) beispielsweise durch Plasmabehandlung oder Beschichtung modifizierte Wandbereiche zur Steuerung des Fließtransports aufweist.

18. Analytisches Testelement nach einem der Ansprüche 1 bis 17, **dadurch gekennzeichnet, dass** die Kanalstruktur (14) insbesondere durch hydrophile oder hydrophobe Kanalabschnitte gebildete Ventilelemente (56) zur Steuerung des Fließtransports aufweist.

19. Analytisches Testelement nach einem der Ansprüche 1 bis 18, **dadurch gekennzeichnet, dass** der Fließtransport in der Kanalstruktur (14) durch auf den Substratkörper (12) einwirkende äußere Steuerungsmittel, insbesondere lokale Druckbeaufschlagung oder Zentrifugalkräfte steuerbar ist.

20. Verfahren zur Durchführung von Blutuntersuchungen, insbesondere als Einmal-Schnelltest, bei welchem eine Blutprobe in einem analytischen Testelement (10) über eine vorzugsweise mikrofluidische Kanalstruktur (14) von einer Aufgabestelle (18) zu mindestens einer Untersuchungsstelle (20,22) geleitet wird, **dadurch gekennzeichnet, dass** aus der Blutprobe Flüssigbestandteile gewonnen und in einen zu analysierenden Teil der Blutprobe zu dessen Verdünnung eingebracht werden.

21. Verfahren nach Anspruch 20, **dadurch gekennzeichnet, dass** eine Vollblutprobe als Ausgangsmaterial in parallelen Teilströmen in einen Verdünnungskanal (24) und einen Probenkanal (26) der Kanalstruktur (14) eingespeist wird, und dass der in dem Verdünnungskanal (24) von Zellbestandteilen abgereicherte Teilstrom an einer Mischstelle (40) mit dem Teilstrom in dem Probenkanal (26) zusammengeführt wird.

## Claims

1. Analytical test element for blood analyses in particular by a single-use rapid test comprising a substrate body (12) having a preferably microfluidic channel structure (14) for the flow transport of a blood sample from an application site (18) to at least one analytical site (20, 22), **characterized in that** the channel structure (14) has a dilution channel (24) that can be loaded with the blood sample and is provided with separation means (36) for retaining corpuscular blood components and has a sample channel (26) which joins the dilution channel (24) at a mixing site (40) and conveys a blood sample aliquot to be diluted.

2. Analytical test element as claimed in claim 1, **characterized in that** the blood sample can be fed into the sample channel (26) and the dilution channel (24) via a junction (32) which divides the sample flow into parallel flows.

3. Analytical test element as claimed in claim 1 or 2, **characterized in that** the channel cross-sections of the sample and dilution channel (26, 24) are adjusted relative to one another to set a predetermined dividing ratio for the subflows of the blood sample that pass through.

4. Analytical test element as claimed in one of the claims 1 to 3, **characterized in that** the flow rate through the dilution channel (24) is more than 10-fold and preferably more than 100-fold higher than the flow rate through the sample channel (26).

5. Analytical test element as claimed in one of the claims 1 to 4, **characterized in that** a filter element consisting in particular of a glass fibre fleece or a microporous filter matrix or filter membrane is disposed as a separation means (36) in the dilution channel (24) and preferably in a filter chamber (34).

6. Analytical test element as claimed in one of the claims 1 to 5, **characterized in that** the dilution channel (24) has a microstructure geometry designed to retain cell components of the blood sample as a separation means (36).

7. Analytical test element as claimed in one of the claims 1 to 6, **characterized in that** the mixing site (40) comprises a lysing chamber (40) provided with a lysing agent (38) to haemolyse the diluted blood sample.

8. Analytical test element as claimed in one of the claims 1 to 7, **characterized in that** the channel structure (14) has a first analytical channel (28) to determine the total haemoglobin value (Hb) of the blood sample and a second analytical channel (30) for determining a glycohaemoglobin value (HbA1c) of the blood sample.

9. Analytical test element as claimed in claim 8, **characterized in that** the analytical channels (28, 30) can be loaded with the diluted blood sample via a branch (41) acting as a flow divider downstream of the mixing site (40).

10. Analytical test element as claimed in claim 8 or 9, **characterized in that** the first analytical channel (28) has an oxidation chamber (44) containing a stored oxidizing agent (42) and in particular ferricyanide to oxidize released haemoglobin.

11. Analytical test element as claimed in one of the claims 8 to 10, **characterized in that** the second analytical channel (30) is designed for the immuno-turbidimetric determination of the glycohaemoglobin concentration.

12. Analytical test element as claimed in one of the claims 8 to 11, **characterized in that** the second analytical channel (30) has a first reaction chamber (46) containing HbA1c antibodies (48) dispensed therein to form soluble antigen-antibody complexes with the glycohaemoglobin from the blood sample.

13. Analytical test element as claimed in claim 12, **characterized in that** the second analytical channel (30) has a second reaction chamber (50) downstream of the first reaction chamber in which an agglutinator (52) is stored to form insoluble immunocomplexes with excess HbA1c antibodies.

14. Analytical test element as claimed in one of the claims 8 to 13, **characterized in that** end sections of the analytical channel (28, 30) designed as cuvettes for a photometric analysis each form one analytical site (20, 22).

15. Analytical test element as claimed in one of the claims 8 to 14, **characterized in that** the analytical channels (28, 30) discharge into a collecting reservoir (54).

16. Analytical test element as claimed in one of the claims 1 to 15, **characterized in that** the channel structure (14) at least in a section thereof has a capillary geometry for an automatic capillary-active flow transport.

17. Analytical test element as claimed in one of the claims 1 to 16, **characterized in that** the channel structure (14) has wall sections for regulating the flow transport that have for example been modified by plasma treatment or coating.

18. Analytical test element as claimed in one of the claims 1 to 17, **characterized in that** the channel structure (14) has valve elements (56) for regulating the flow transport that are formed in particular by hydrophilic or hydrophobic channel sections.

19. Analytical test element as claimed in one of the claims 1 to 18, **characterized in that** the flow transport in the channel structure (14) can be regulated by external control means acting on the substrate body (12) and especially by local application of pressure or centrifugal forces.

20. Method for carrying out blood analyses in particular as a single-use rapid test in which a blood sample is conveyed in an analytical test element (10) via a preferably microfluidic channel structure (14) from an application site (18) to at least one analytical site (20, 22), **characterized in that** liquid components are obtained from the blood sample and added to a portion of the blood sample to be analysed in order to dilute it.

21. Method as claimed in claim 20, **characterized in that** a whole blood sample as the starting material is fed in parallel subflows into a dilution channel (24) and a sample channel (26) of the channel structure (14) and the subflow that has been depleted of cell components in the dilution channel (24) is joined with the subflow in the sample channel (26) at a mixing site (40).

## Revendications

1. Elément d'essai analytique pour test sanguin, en particulier par un essai rapide unique, avec un corps de substrat (12) présentant une structure de canal (14), de préférence microfluidique, pour le transport par écoulement d'une prise de sang depuis un point d'alimentation (18) jusqu'à un point d'analyse (20, 22), **caractérisé en ce que** la structure de canal (14) présente un canal de dilution (24) pouvant être alimenté avec la prise de sang, pourvu de moyens séparateurs (36) destinés à retenir les constituants sanguins corpusculaires, et un canal d'échantillon (26) réuni avec le canal de dilution (24) au niveau d'un point de mélange (40), amenant un aliquote de prise de sang à diluer.

2. Elément d'essai analytique selon la revendication 1, **caractérisé en ce que** le canal d'échantillon (26) et le canal de dilution (24) peuvent être alimentés avec la prise de sang via un noeud (32) avec séparation parallèle du flux d'échantillon.

3. Elément d'essai analytique selon la revendication 1 ou 2, **caractérisé en ce que** les sections transversales du canal d'échantillon et de dilution (26, 24) sont coordonnées l'une à l'autre pour régler un rapport de séparation prédéterminé des flux partiels guidés de la prise de sang.

4. Elément d'essai analytique selon l'une quelconque des revendications 1 à 3, **caractérisé en ce que** le débit à travers le canal de dilution (24) est 10 fois supérieur, de préférence 100 fois supérieur au débit à travers le canal d'échantillon (26).

5. Elément d'essai analytique selon l'une quelconque des revendications 1 à 4, **caractérisé en ce que** dans le canal de dilution (24), de préférence dans une chambre de filtration (34), est disposé comme moyen séparateur (36) un élément de filtration formé en particulier par un non-tissé de fibres de verre ou une matrice de filtration ou membrane de filtration microporeuse.

6. Elément d'essai analytique selon l'une quelconque des revendications 1 à 5, **caractérisé en ce que** le canal de dilution (24) possède comme moyen séparateur (36) une géométrie microstructurale formée pour retenir les constituants cellulaires de la prise de sang.

7. Elément d'essai analytique selon l'une quelconque des revendications 1 à 6, **caractérisé en ce que** le point de mélange (40) est formé par une chambre de lyse (40) pourvue d'un agent de lyse (38) en vue d'une hémolyse de la prise de sang diluée.

8. Elément d'essai analytique selon l'une quelconque des revendications 1 à 7, **caractérisé en ce que** la structure de canal (14) présente un premier canal d'analyse (28) pour déterminer la valeur totale d'hémoglobine (Hb) de la prise de sang, et un second canal d'analyse (30) pour déterminer une valeur total d'hémoglobine glycosylée (HbA1c) de la prise de sang.

9. Elément d'essai analytique selon la revendication 8, **caractérisé en ce que** les canaux d'analyse (28, 30) peuvent être alimentés avec la prise de sang diluée via une ramification (41) en tant que répartiteur de flux en aval du point de mélange (40).

10. Elément d'essai analytique selon la revendication 8 ou 9, **caractérisé en ce que** le premier canal d'analyse (28) présente une chambre d'oxydation (44) avec un agent d'oxydation stocké (42), en particulier du ferricyanure pour l'oxydation de l'hémoglobine libérée.

11. Elément d'essai analytique selon l'une quelconque des revendications 8 à 10, **caractérisé en ce que** le second canal d'analyse (30) est formé pour déterminer par immunoturbidimétrie la concentration d'hémoglobine glycosylée.

12. Elément d'essai analytique selon l'une quelconque des revendications 8 à 11, **caractérisé en ce que** le second canal d'analyse (30) présente une première chambre de réaction (46) avec des anticorps HbA1c (48) dispersés à l'intérieur afin de former des complexes solubles antigène-anticorps avec l'hémoglobine glycosylée issue de la prise de sang.

13. Elément d'essai analytique selon la revendication 12, **caractérisé en ce que** le second canal d'analyse (30) présente une seconde chambre de réaction (50) située en aval de la première chambre de réaction avec un agglutinateur stocké (52) pour former des complexes immuns insolubles avec des anticorps HbA1c excédentaires.

14. Elément d'essai analytique selon l'une quelconque des revendications 8 à 13, **caractérisé en ce que** les sections terminales des canaux d'analyse (28, 30) conçues comme cuvettes pour un test photométrique forment respectivement un point d'analyse (20, 22).

15. Elément d'essai analytique selon l'une quelconque des revendications 8 à 14, **caractérisé en ce que** les canaux d'analyse (28, 30) débouchent dans un réservoir collecteur (54).

16. Elément d'essai analytique selon l'une quelconque des revendications 1 à 15, **caractérisé en ce que** la structure de canal (14) présente au moins par sections une géométrie capillaire pour un transport tensioactif automatique par écoulement.

17. Elément d'essai analytique selon l'une quelconque des revendications 1 à 16, **caractérisé en ce que** la structure de canal (14) présente des zones pariétales modifiées par exemple par traitement plasma ou revêtement afin de commander le transport par écoulement.

18. Elément d'essai analytique selon l'une quelconque des revendications 1 à 17, **caractérisé en ce que** la structure de canal (14) présente des éléments de vanne (56) formés en particulier par des sections de canal hydrophiles ou hydrophobes afin de commander le transport par écoulement.

19. Elément d'essai analytique selon l'une quelconque des revendications 1 à 18, **caractérisé en ce que** le transport par écoulement dans la structure de canal (14) peut être commandé par des moyens extérieurs de commande, en particulier des pressurisations locales ou des forces centrifuges, agissant sur le corps de substrat (12).

20. Procédé pour mettre en oeuvre des tests sanguins, en particulier comme essai rapide unique, dans lequel une prise de sang est guidée dans un élément d'essai analytique (10) via une structure de canal (14) de préférence microfluidique depuis un point d'alimentation (18) jusqu'à au moins un point d'analyse (20, 22), **caractérisé en ce que** des constituants liquides sont extraits de la prise de sang et sont introduits dans une partie à analyser de la prise de sang en vue de la dilution de celle-ci.

21. Procédé selon la revendication 20, **caractérisé en ce qu'**une prise de sang complet est injecté comme produit de base dans des flux partiels parallèles dans un canal de dilution (24) et dans un canal d'échantillon (26) de la structure de canal (14), et **en ce que** le flux partiel appauvri en constituants cellulaires dans le canal de dilution (24) est réuni avec le flux partiel dans le canal d'échantillon (26) au niveau d'un point de mélange (40).
